# EUROPEAN PATENT APPLICATION

(11) **EP 1 393 636 A1**
(43) Date of publication of application: **03.03.2004**
(21) Application number: 02731036.6
(22) Date of filing: 14.05.2002
(51) Int. Cl.: A23L 1/30, A23L 1/305, A23L 1/304, A23L 1/29, A23J 1/00, A23K 1/175, A23K 1/16

(54) **BIOLOGICALLY ACTIVE FOOD ADDITIVE AND BIOLOGICALLY ACTIVE FORAGE ADDITIVE FOR PREVENTING IODINE DEFICIENCY AND OPTIMISING IODINE METABOLISM, FOOD AND FORAGE PRODUCTS CONTAINING SAID ADDITIVES**

(30) Priority: 14.05.2001 RU 2001112481; 18.02.2002 RU 2002103956
(71) Applicant: Andreichuk, Vasily Petrovich, Borovsk, 249010 (RU)
(72) Inventor: ANDREICHUK, Vasily Petrovich, Kaluzhskaya obl., 249010 (RU); ANDREICHUK, Elena Viktorovna, Kaluzhskaya obl., 249010 (RU); ANDREICHUK, Dmitry Vasilievich, Kaluzhskaya obl., 249010 (RU); TIGRANYAN, Ruben Ambartsumovich, Moscow, 125212 (RU)
(74) Representative: HOFFMANN EITLE
(86) International application number: PCT/RU2002/000230
(87) International publication number: WO 2002/091860

(57) **Abstract**

The invention relates to the food industry and agriculture and is directed to prevent iodine deficiency and optimise iodine metabolism in a human and animal organism. The inventive biologically active food additive (BAA) and food products, the biologically active forage additive (BAFA) and forage products comprise synthetic organic compounds containing covalently linked iodine selected from the following group: carboxylic acids, unsaturated fat acids, lipids, terpens, alkanes, terpenoids, isoprenes, peptides, polypeptides, amino acids, protein hydrolisates, polypeptide hydrolisates, vegetable, animal and microbiological proteins, mixture of lipids and unsaturated fat acids, mixture of isoprenes and terpens, mixture of isoprenes and protein hydrolisates, mixture of isoprenes and unsaturated fat acids, mixture of vegetable, animal and microbiological proteins, mixture of vegetable and animal proteins, mixture of animal and microbiological proteins, mixture of vegetable and microbiological proteins. Iodine is also covalently linked in the 5^{th} and 3^{rd} or in the 3^{rd} position of the phenolic cycle in proteins, peptides, polypeptides, aminoacids and in polypeptide and protein hydrolisates. The aminoacids and proteins are selected from a group of aminoacids and proteins devoid of hormonal and thyroid activity and produced in conditions, which exclude the condensation of tyrosine nucleus.

## Description

### Technical field and background of the invention

The invention relates to the food industry and agriculture, namely to the preparation of bioactive additives to food and prophylactic food products containing BAAs and also to the production of bioactive food additives (BAFA) and feeds containing same or - to be more precise - the present invention is directed to the prophylaxis of iodine deficiency and optimization of iodine metabolism in human beings and animals.

Iodine is a structurant of thyroid hormones being involved in performing their biological function - growth regulation and tissue differentiation, control over metabolism and energy, thermal production. Despite a negligible need in this vital microelement, the living organisms, humans and animals included, always experience iodine deficit to this or that extent, which is characteristic of extensive territories.

The significance and indispensability of iodine for normal development and functioning of the living organisms stimulate the artificial enrichment of foodstuffs and food products with this microelement.

Known is iodized common salt representing a mixture of sodium chloride and iodine inorganic compounds to be used for removing iodine deficiency in human ration. Its defect is complexity of providing fixed iodine consumption because of the technological difficulties of preparing qualitative iodized salt and individual variability of its consumption. With the consumption of this salt, there are observed a growing number of hyperthyreosis cases. The mineral compounds of iodine-potassium iodate and potassium iodide used for iodating the salt and other foodstuffs perform the function of an additional iodine source under natural conditions. And from the viewpoint of evolution, an iodine metabolism mechanism, as such, is directed, first of all, to using organic iodine form being predominant in natural food products (say, animal and plant origin products, sea kale).

Also known is the use, as an iodine source, or organic compounds-amyloiodine and iodinol. A disadvantage of these preparation is a non-covalent bond of iodine with an organic carrier and, as this is so, these agents behave in an organism as sources of mineral iodine.

Further known is a preparation for the prevention and treatment of diseases of the thyroid gland, which is an organic compound with covalent bound iodine- thyroidin iodized protein. However, said thyroidin has hormonal activity. Administration of hormonal preparations should be carried out under stringent medical control to take account of concomitant diseases. Wrong dosage causes serious complications. The use of said thyroidin, as an iodine source, is impermissible as leading to replacing part of natural hormones with those from without. Responsive to this, the thyroid gland lowers its physiological activity and diminishes in size down to atrophy. And a further overdosage of the preparation is likely to cause artificial hyperthyreosis (The Great Medical Encyclopaedia, Moscow, the Soviet Encyclopaedia, 1963, pp.202-203).

The most pertinent prior art solution to the first two subject-matters of invention is a bioactive additive to food for the preventive treatment of iodine deficiency, which contains an organic iodine-containing agent, and a foodstuff containing this bioactive additive (Patent RF No 2134520, cl. A 23 J 1/00, 1998).

Disadvantages of known analogs are relatively low efficiency thereof and unacceptability for adequately influencing an organism for the purpose of diminishing or removing the physiologo-biochemical disturbances of iodine metabolism.

For iodine metabolism disturbances to be prevented, currently practiced are the use of compounds based on inorganic iodine and iodination by said compounds, of foodstuffs to bring its daily consumption to physiological norms; the use of organic iodine form prevailing in natural food products (for example, in sea kale); the intake of BAA to food on the basis of plant iodine sources and also the application of iodine-containing hormonal preparations.

Known in the art is the use of inorganic iodine, for example a potassium iodate or potassium iodide, for the removal of iodine deficiency in an animal body by way of including same in feed mixes and food additives (cf. patent RU 2041647, cl. A23K 1/16, 20.08.1995 and patent RU 2164758, cl. A23K 1/16, 10.04.2001).

A disadvantage of such feeds and additives is complexity of providing normalized iodine consumption owing to the technical difficulties in preparing such iodine-containing feeds and additives. Also, mineral iodine compounds are not physiologically adequate iodine form for an organism and represent under natural conditions only as an additional iodine source, as distinct from organic iodine-containing substances.

Known are some food additives and feeds obtained on the basis of various sea algae - laminaria (cf. patent RU 2134522, cl. A23L 1/30, 20.08.1999), on the basis of laminaria, chlorella and spirulina (cf. patent RU 2131198, cl. A23K 1/00, 10.06.1999). However, the preparation of such additives and feeds calls for special technology.

Known in the art is a preparation containing iodine in organically bound form-iodocasein-iodized protein (cf. Physiology of farm animals. (Under the editorship of N.A. Shmanenkov. Leningrad, NAUKA Publishers, 1978, pp. 505-506). A serious defect of this preparation is the fact that it was used in animal husbandry as a hormonal preparation for regulating productivity level. More, be it noted that administration of hormonal preparations should be carried out under strict veterinary control to take account of concomitant diseases. Wrong dosage results in grave complications.

The most pertinent prior art solution to another two subject-matters of invention is a bioactive food additive for the preventive treatment of iodine deficiency, containing an organic iodine- containing agent, and a food product containing this bioactive food additive (patent RU No 2086147, cl. A23K 1/00, 1997).

Disadvantages of known analogs are their relatively low efficiency, unacceptability for exerting an adequate influence on animals to diminish or to remove the physiologo-biochemical iodine metabolism abnormalities.

With that so, one can make the conclusion that for the preventive treatment of iodine metabolism abnormalities in animals currently practiced is the use of inorganic iodine-based compounds, as is iodination of feeds, using said compounds, to bring a daily consumption of iodine to physiological norms; feeding an organic iodine form prevailing in natural plant feeds (for example, in sea kale) and also administration of iodine-containing hormonal preparations into animals.

### Summary of the invention

The present invention is directed to not only solving the problem of the prophylaxis of iodine in short supply but also to removing iodine metabolism abnormalities in humans and animals in general. The task is to create BAA to food and foodstuffs as well as BAFA and food products containing the most physiologically essential iodine- containing compounds of organic origin, having no hormonal activity, being adequate to the biochemical peculiarities of an iodine metabolism course and, therefore, being capable of providing for triggering mechanisms of self-regulation and automatic control thereof. And the obtainable compounds should take account of the individual specific features of iodine metabolism in the living organism when employed.

This task is solved owing to the fact that a bioactive additive to food (BAA to food) for the prevention of iodine failure includes an organic iodine-containing agent. According to the invention, the organic iodine-containing agent represents an organic synthetic compound with covalent-bound iodine selected from the group consisting of carboxylic acids, unsaturated fatty acids, lipids, terpenes, alkanes, terpenoids, isoprenes, peptides, polypeptides, amino acids, protein hydrolyzates, polypeptide hydrolyzates, proteins of plant /animal/microbiological origin, a mixture of lipids and unsaturated fatty acids, a mixture of isoprenes and terpenes, a mixture of isoprenes and protein hydrolyzates, a mixture of isoprenes and unsaturated fatty acids, a mixture of proteins of plant, animal and microbiological origin, a mixture of proteins of plant and animal origin, a mixture of proteins of animal and microbiological origin, a mixture of proteins of plant and microbiological origin, and in the proteins, peptides, polypeptides, amino acids, polypeptide and protein hydrolyzates, the iodine is covalent bound at the 5- and 3-positions or at the 3-position of the phenolic ring and, more importantly, the amino acids and proteins are selected from the group of amino acids and proteins, having no hormonal thyroid activity, to be obtained under conditions precluding the condensation of tyrosine kernels.

It can be to advantage to use caseins, egg albumin, lactic or whey proteins and hemoglobins as animal proteins; soy proteins as plant proteins; yeast proteins (yeast hydrolyzate) as microbiological proteins.

In addition, BAA to food may contain an organic compound with non-covalent bound iodine and/or inorganic iodine. BAA to food is desired to have the sustained form of tablets, powders, capsules, microcapsules, dragee, solutions, and the microcapsules are obtained by way of microincapsulation, using carbohydrate, protein or lipid raw material.

Iodine-containing material may be within an inert polymer matrix from, for example, carrageenan, pectin, agarose or uronic acids.

BAA to food may be within vitamin - mineral complexes.

A second subject-matter of invention is a foodstuff containing the above-mentioned BAA to food according to any one of the afore-said combinations.

The foodstuff is a milk product, lactic acid product, meat product, bakery product, bread, beverage, juice, confectionery, infant formula product.

A third subject-matter of invention is a bioactive food additive (BAFA) for the prophylaxis of iodine failure, which contains an organic iodine-containing agent. According to the invention, the organic iodine-containing agent is an organic synthetic compound with covalent bound iodine, selected from the group consisting of carboxylic acids, unsaturated fatty acids, lipis, terpenes, alkanes, terpenoids, isoprenes, peptides, polypeptides, amino acids, protein hydrolyzates, polypeptide hydrolyzates, proteins of plant/animal/microbiological origin, a mixture of lipids and unsaturated fatty acids, a mixture of isoprenes and terpenes, a mixture of isoprenes and protein hydrolyzates, a mixture of isoprenes and unsaturated fatty acids, a mixture of proteins of plant, animal and microbiological origin, a mixture of proteins of plant and animal origin, a mixture of proteins of animal and microbiological origin, a mixture of proteins of plant and microbiological origin, and in the proteins, peptides, polypeptides, amino acids, polypeptide and protein hydrolyzates, the iodine is covalent bound at the 5- and 3-positions or at the 3-position of the phenolic ring, the amino acids and proteins being selected from the group of amino acids and proteins, having no hormonal thyroid activity, and being obtained under conditions precluding the condensation of tyrosine kernels.

It is advisable to use, as animal proteins, caseins, egg albumin, lactic or whey proteins, hemoglobins; as vegetable proteins - soy proteins; as microbiological proteins - for example, yeast proteins as an yeast hydrolyzate.

In addition, BAFA may comprise an organic compound with non-covalent bound iodine and/or inorganic iodine.

BAFA may represent prolonged form and be advantageously implemented as tablets, powders, capsules, microcapsules, dragee, solutions, injectable solutions and, along with this, the microcapsules are obtained by way of microincapsulation with the use of carbonhydrate, protein or lipid raw material.

Iodine-containing material may be included in the composition of an inert polymer matrix, say, from carrageenan, pectin, agarose or uronic acids.

BAFA may be within vitamin-mineral complexes, premixes, feeds or represent a ready-to-use premix.

A fourth subject-matter of invention is a food product containing the above-mentioned BAFA according to any one of said combinations.

### Detailed description of essence of the invention

The compounds offered for use in the present invention are as follows: proteins, peptides and polypeptides. We have separated out these notions belonging to various groups of substances, on the basis of definitions as given below:

Peptides are organic compounds consisting of two or more amino-acid residues being bound by way of peptides, which can also be obtained as products of an incomplete hydrolysis of polypeptides or proteins. Peptides comprising more than 10 amino acid residues are called polypeptides (A. Lenindjer, Fundamentals of Biochemistry, Moscow, MIR Publishers, 1985, page 132).

Proteins are high-molecular nitrogen-containing organic compounds and represent polypeptide chains containing from 100 to 1000 and more of amino-acid monomeric units being bound one with another by way of peptides (ibidum, page 160).

The proposed organic iodine-containing compounds within BAA to food, foodstuffs, BAFAs and food products have no hormonal activity; these contain iodine is covalent bound form permitting using these substances both as iodine sources and substrates, which makes it possible to optimize iodine metabolism in general.

As stated above, an alternative embodiment of the invention can be accomplished in several combinations. BAAs to food and foodstuffs accordingly, BAFAs and food products accordingly, may contain both one of the synthetic organic compounds with covalent bound iodine from the group consisting of carboxylic acids, unsaturated fatty acids, lipids, terpenes, alkanes, terpenoids, isoprenes, peptides, polypeptides, amino acids, protein hydrolyzates, polypeptide hydrolyzates, proteins of plant, animal and microbiological origin and a mixture of synthetic organic compounds with covalent bound iodine from the group comprising a mixture of lipids and unsaturated fatty acids, a mixture of isoprenes and terpenes, a mixture of isoprenes and protein hydrolyzates, a mixture of isoprenes and unsaturated fatty acids, a mixture of proteins of plant, animal and microbiological origin, a mixture of proteins of plant and animal origin, a mixture of proteins of animal and microbiological origin, a mixture of proteins of plant and microbiological origin. Also, there must be observed the following condition: in the proteins, peptides, polypeptides, amino acids, polypeptide and protein hydrolyzates, the iodine is covalent bound at the 5-and 3-positions or at the 3-position of the phenolic ring while the amino acids and proteins should be selected from the group of amino acids and proteins having no hormonal thyroid activity, which are obtained under conditions precluding the condensation of tyrosine kernels.

As so, if we are to enumerate in detail all the combinations of an alternative embodiment of the invention, then BAAs to food and foodstuffs accordingly, BAFAs and food products accordingly, may contain one of the following compounds:
peptides in which iodine is covalent bound at the 5- and 3- positions of the phenolic ring, peptides in which iodine is covalent bound at the 3-position of the phenolic ring,
polypeptides in which iodine is covalent bound at the 5- and 3- positions of the phenolic ring, polypeptide in which iodine is covalent bound at the 3-position of the phenolic ring,
polypeptide hydrolyzates in which iodine is covalent bound at the 5- and 3-positions of the phenolic ring, polypeptide hydrolyzates in which iodine is covalent bound at the 3-position of the phenolic ring,
amino acids in which iodine is covalent bound at the 5- and 3- positions of the phenolic ring, amino acids in which iodine is covalent bound at the 3-position of the phenolic ring, and, along with this, these amino acids are selected from the group of amino acids having no hormonal thyroid activity, which are obtained under conditions precluding the condensation of tyrosine kernels,
protein hydrolyzates in which iodine is covalent bound at the 5- and 3-positions of the phenolic ring, protein hydrolyzates in which iodine is covalent bound at the 3-positions of the phenolic ring,
proteins of plant origin in which iodine is covalent bound at the 5- and 3-positions of the phenolic ring, proteins of plant origin in which iodine is covalent bound at the 3-position of the phenolic ring, and said proteins are selected from the group of proteins having no hormonal thyroid activity and are obtained under conditions precluding the condensation of tyrosine kernels,
proteins of the animal origin in which iodine is covalent bound at the 5- and 3-positions of the phenolic ring, proteins of animal origin in which iodine is covalent bound at the 3-position of the phenolic ring and these proteins are selected from the group of proteins having no hormonal thyroid activity and are obtained under conditions precluding the condensation of tyrosine kernels,
proteins of microbiological origin in which iodine is covalent bound at the 5- and 3- positions of the phenolic ring, proteins of microbiological origin in which iodine is covalent bound at the 3-position of the phenolic ring, said proteins being selected from the group of proteins, having no hormonal thyroid activity, and being obtained under conditions precluding the condensation of tyrosine kernels,
a mixture of isoprenes and protein hydrolyzates in which iodine is covalent bound at the 5- and 3- positions of the phenolic ring, a mixture of isoprenes and protein hydrolyzates in which iodine is covalent bound at the 3- position of the phenolic ring,
a mixture of proteins of plant, animal and microbiological origin in which iodine is covalent bound at the 5- and 3- positions of the phenolic ring, a mixture of proteins of plant, animal and microbiological origin in which iodine is covalent bound at the 3-position of the phenolic ring, said proteins being selected from the group of proteins having no hormonal thyroid activity, and being obtained under conditions precluding the condensation of tyrosine kernels,
a mixture of proteins of plant and animal origin in which iodine is covalent bound at the 5- and 3- positions of the phenolic ring, a mixture of proteins of plant and animal origin in which iodine is covalent bound at the 3- position of the phenolic ring and these proteins are selected from the group of proteins having no hormonal thyroid activity and are obtained under conditions precluding the condensation of tyrosine kernels,
a mixture of proteins of animal and microbiological origin in which iodine is covalent bound at the 5- and 3- positions of the phenolic ring, a mixture of proteins of animal and microbiological origin, in which iodine is covalent bound at the 3- position of the phenolic ring, said proteins being selected from the group of proteins having no hormonal thyroid activity, and being obtained under conditions precluding the condensation of tyrosine kernels,
a mixture of proteins of plant and microbiological origin in which iodine is covalent bound at the 5- and 3-positions of the phenolic ring, a mixture of proteins of plant and microbiological origin in which iodine is covalent bound at the 3-position of the phenolic ring, and these proteins are selected from the group of proteins having no hormonal thyroid activity and are obtained under conditions precluding the condensation of tyrosine kernels.

For the production of BAA to food and prophylactic foodstuffs and also DAFA and food products there are proposed the combined and sustained forms of iodine-containing compounds providing for an individual approach to the preventive treatment of diseases associated with iodine metabolism malfunctions.

Intestine epithelium effects the differentiated suction of iodine-suction of iodine and the transmembrane transfer of organic compounds with covalent bound iodine. In the latter instance, the iodine is sucked as a single complex with an organic carrier without a preliminary relaxation in the bowels. Subsequently the iodide from the intestine gets into the blood and is accumulated by the thyroid while the covalent bound organic iodine becomes accessible to tissue metabolism and acquires the possibility to be deposited not in the thyroid alone. The main role in the further metabolism of iodorganic compounds is to be played by the liver, wherein the fermentative separation of chemically bound iodine occurs in the form of an iodide ion to be withdrawn by the thyroid from the blood for synthetic needs thereof. Along with deposition in the liver there takes place the conjugation of iodine-containing metabolites with glucuronic acid to be subsequently excreted with the urine or bile. The iodinated amino acids are subjected in the liver to reamination, decarboxylation, hydroxylation, oxidizing deamination, and methylation. Iodine-containing pyruvate and acetate analogs can be excreted with the urine. Thus, the iodine-containing compounds realize practically all the metabolic possibilities of iodine metabolism, which in turn provides an additional ability of an organism for the self-regulation and automatic control of this process.

Iodine suction in the intestine is a key factors of maintaining its homeostasis in an organism and is the result of both a physiological condition of the intestine and a functional condition of suction biochemical mechanisms. The claimed invention enables one to directly exert an influence on these processes, using various classes of iodine-containing preparations. For example, at the time of sucking an iodinated amino acid an iodinated unsaturated fatty acid various mechanisms are enabled for transmembrane transfer and further tissue metabolism thereof. Inasmuch as tissue dehalogenases with varying efficiency perform the deiodination of different iodorganic substrates, this makes possible regulation of iodine metabolism at the stage of separation of the iodine from an organic component and its inflow into a blood canal and also at the level of peripheral metabolism. It is proposed that various forms of iodine-containing preparations should be put to use, which will not only provide the organism with iodine but also will optimize iodine metabolism on the strength of specific features of metabolism thereof.

It is desired to use a preparation in the form of simple, combined and sustained (prolonged) forms. The simple form represents a synthetic organic compound of a non-hormonal nature which contains covalent bound iodine. Iodine-containing synthetic agents are obtained by way of iodination of the organic compounds of different classes, say, a phenolic derivative, such as tyrosine, both in a free state and within proteins, histidine amino acid or its amino acid residue, derivatives of a terpene, terpenoid or isoprene nature, unsaturated fatty acids, alkanes, lipids, proteins or peptides, polypeptides, protein or polypeptide hydrolyzates as well as the mixtures of some compounds thus far indicated. Iodination of organic substances is carried out by a chemical or fermentative way or simultaneously by a chemical and fermentative method or by a biotechnological method with the use of prior art methods for the purpose (cf.L.A.Osterman. Examination of biological macromolecules. Moscow, NAUKA Publishers, 1983, page 244; L.P. Hager "Iodination of Tyrozine by Chloroperoxidase", In "Methods of Enzymology", v.XVII, Part A. Acad.Press, N.Y.,London, 1970; Morrison M. "Iodination of Tyrozine: Isolation of Lactoperoxidase", In "Methods in Enzymology", v. XVII, Part A, Acad. Press, N.Y., London, 1970; Missouri. Agric. Exp. Stat., Research Bull, v. 355, 1942; Hunter W.M., Greenwood F.C., Nature, London, v. 194, 1962, page 495).

With that so, amino acids and proteins are selected from the group of amino acids and proteins having no hormonal thyroid activity, which are obtained under conditions precluding the condensation of tyrosine kernels.

It is advisable to conduct iodination of peptides, polypeptides, proteins in terms of tyrosine or histidine amino acid residues or simultaneously. It can be advantageous to obtain a synthetic organic agent with covalent bound iodine by the hydrolysis of preiodized proteins or polypeptides.

It is useful to utilize, as an organic agent with covalent bound iodine, iodinated unsaturated fatty acids in a free or chemically bound state or in combination of free fatty acids and chemical compounds thereof.

It is also useful to manufacture the combined forms of iodine-containing BAAs and BAFAs, which represent a combination of organic iodine in covalent form and organic iodine in non-covalent form as well as organic iodine in covalent form and inorganic iodine. Such BAAs and BAFAs provide for activating the alternative mechanisms of iodine suction and are required in cases of iodine metabolism disturbed at this stage.

It is desired to use the substained form of an iodine-containing agent-synthetic organic compound with covalent bound iodine within an inert polymer matrix having a three-dimentional molecular sieve structure or within a microcapsule (microsphere) of a protein, lipid or carbohydrate nature. The polymer matrix or microcapsule (microsphere) causes the sterical hindrances of iodorganic matter uptake, prolonging this process and, consequently, a suction process. The sterical hindrances for proteolysis of the polymer-iccorporated iodinated proteins or peptides also prolong iodine-containing amino acid uptake and suction thereof. The plononged form of iodine-containing BAA and BAFA may be represented by a combination of iodine-containing compounds of a different character - combination of inorganic, covalent and non-covalent bound iodine in the composition of organic compounds. For the polymer matrix to be obtained, use can be made of agaroses, pectins, alginic acids, carrageenans, uronic acids. For the purpose of prolongation, the microcapsules are obtained by way of microincapsulation using carbohydrate, protein or lipid raw material and, more importantly, the prolonged form of an iodine-containing agent may be the mixtures of synthetic iodine-containing substances of a different chemical nature as thus far mentioned.

It is desired to use BAFA in the form of an injectable solution to provide for iodine to enter the thyroid gland of an animal during several months with the absence of a regular feeding-up with iodine-containing additives.

Consumption of all the forms of iodine-containing compounds for human beings is regulated in accordance with the corresponding iodine-established prophylactic, physiological and age-dependent limits and norms. Administration of synthetic, covalent bound, iodinated organic compounds into a human body is carried out in the composition of bioactive additives to food in various forms, vitamin-mineral complexes and variegated foodstuffs. Enrichment of food products is carried out with the use of iodorganic matter in dry form in the form of a solution.

Consumption of all the forms of iodine-containing compounds for animals is regulated in keeping with the corresponding iodine-established veterinary, specific and prophylactic limits and norms. Administration of covalent bound, synthetic, iodinated organic compounds into an animal body is effected within bioactive food additives in various forms, vitamin-mineral premixes and different food products. Enrichment of feeds is effected with the use of iodorganic matter in dry form or in the form of a solution.

Synthetic organic substances containing covalent bound iodine display chemical stability, thermolight resistance, good solubility in water or organic solvents. The physicochemical properties of these compounds permit utilizing same to enrich a wide range of feeds for animals and a wide variety of foodstuffs - milk and lactic acid, meat, bread and bakery products, confectionery, beverages, juices, child's food and so on, and so forth.

Inasmuch as the proposed iodination methods and organic substances selected for the purpose, of a different chemical nature assure a high degree of iodination, then the thus obtained iodine-containing substances are used in microquantities and, most importantly, their impact is excluded on the organoleptic properties of enriched products.

The technical result of the invention is an increased efficiency of the preventive treatment of iodine deficiency, enlargement of a range of prophylactic agents in iodine metabolism abnormalities, the possibility to make iodine metabolism the most favourable to take account of the peculiarities of metabolism of iodine-containing compounds in humans and animals, provision of a possibility of an individual approach to the prevention of iodine metabolism abnormalities on account of the directed regulation of this process. Administration of synthetic organic substances, as an iodine source, which contain iodine in covalent bound form into a human body in the form of BAA or enriched foodstuffs and into an animal body in the form of BAFA or enriched food products provides iodine suction as a common part with an organic carrier without the preliminary separation in the gastrointestinal tract, of free iodine or iodide. The non-hormonal organic iodine compounds undergo various metabolic transformations characteristic of iodine metabolism. An iodide ion released as a result of fermentative catalysis enters the thyroid gland. The iodinated synthetic, organic compounds ofa different chemical nature, such as unsaturated fatty acids, amino acids, alkanes, lipids, compounds of an isoprene or terpene nature are sucked with a different degree of efficieny by means of specific, transmembrane transport systems. A choice of the nature of the iodinated synthetic, organic compound along with the regulation of its suction allows one to control the intensity and efficiency of a process of tissue fermentative iodine release from its composition, judging by distinctions in the substrate specificity of various iodine-containing chemical substances, a degree of their accessibility to a fermentative catalysis as a result of the specific tissue deposition of various iodorganic representatives. The biochemical specific features of metabolism of iodinated organic substances represented by the compounds of a different chemical nature provide the possibility to modulate the intensity of individual chemical reactions, exert an influence on the level of kidney or bowels excretion of iodine-containing metabolites. Susceptibility of the iodine-containing synthetic, organic compounds to various metabolic transformations and processes makes it possible to ensure the most favourable iodine metabolism without provoking the rapid changes of iodine concentrations in a blood plasma and also taking account of the individual physiologo-biochemical peculiarities of the organism. The use of the covalent bound organic form of iodine, as the latter's source, made it possible to trigger off the mechanisms of self-regulation and automatic control of iodine metabolism in human and animal organisms.

At the same time, it should be understandable to a person skilled in the art that the manufacture of BAAs and foodstuffs, BAFAs and feeds, based on said synthetic iodine-containing, organic compounds makes it possible to create extensive and inexpensive production lines and facilities in comparison with the manufacture of similar products on the basis of a natural iodine source. A broad raw material base permits adapting the manufacture of BAAs and foodstuffs and also BAFAs and feeds to a concrete technological process and, along with this, the synthetic BAAs and BAFAs of the invention are an equivalent substitution for the natural ones and also cheap and effective for mass consumption.

### Specific embodiments of the invention

The invention is illustrated by the following examples which, however, do not cover and, what is more, limit the entire scope of monopoly sought.

### Example I

A biologically active additive to food for the prophylaxis of iodine failure and optimization of iodine metabolism is prepared by iodination of a food protein - casein rich in tyrosine amino acid residues.

To begin with, protein iodination is carried out in terms of tyrosine amino acid residues by substituting hydrogen atoms with iodine at the 5- and 3-positions of the phenolic ring. Said iodination is effected under conditions precluding the condensation of tyrosine phenolic kernels with the possibility to form iodinated tyronines, which triidotyronine and tetraiodotyronine belong to - compounds having thyroid hormonal activity. A degree of protein iodination is also regulated by reaction conditions. The high degree of iodination (above 8%) enables one to use a biologically active additive to enrich foodstuffs in little amounts. To provide for 100 mcg iodine to be admitted to a human body, it is necessary to introduce about 1.5 mg iodinated protein into 250 g of bread. Such a meager quantity of the preparation does not affect the organoleptic properties of foodstuffs.

Cow milk (120 l) is degreased by separation and concentrated three times by ultrafiltration on roll filters, threshold 10 kDa. A casein fraction of protein is isolated by souring up to pH 4.6. The resulting casein (about 3 kg) is dissolved, using a solution of sodium hydroxide. The protein solution is placed in a temperature-controlled reaction vessel equipped with a stirring apparatus. For iodination, use is made of elementary iodine - fine crystalline and quick-soluble (1-1.2 kg per 3 kg of protein). Control of the temperature of 35-40°C is carried out during the reaction, as is a pH of from 8.0 to 9.5 and the degree of iodination as required. Upon completion ofthe reaction, the iodinated caseins are purified from the inorganic form of iodine. Iodide is removed by reprecipitation of the protein through centrifugation and ultrafiltration. Diafiltration is conducted on the roll filters, threashold 10 kDa, to achieve a final iodide content in the total iodine in the preparation of less than 0.5%. The iodination process provides the highly efficient antiseptic treatment of a solution of lactic proteins.

Purified iodinated proteins are preserved with sucrose (50%) or glycerol (50%). The solution is further sterilized by heating (3-5 minutes) up to 90°. The iodinated protein solution - BAA to food is packed up in glass cups and sealed in vacuo. A guarantee period of storage of the preparation is I to 1.5 years.

The resultant solution of iodinated casein is used for enriching bread. The protein solution is added to a batch before introducing the flour thereby to achieve a uniform distribution of the iodinated protein in the dough and ready-for-use products.

Iodine is bound with tyrosine amino acid residues by a covalent bond. These compounds feature thermal stability. Decomposition of iodotyrosines occurs at a temperature between 199 and 201°. Thus, the technological process of bread baking does not affect the chemical stability of an organic compound with covalent bound iodine in spite of rather high temperature.

An amount of iodinated casein added to bread depends on the degree of endemicity of a region, average bread consumption per head, the degree of iodination of the protein itself.

The iodinated protein in the gastrointestinal tract is hydrolyzed to free amino acids. Iodotyrosines are sucked by the mucosa of the intestine, as a single chemical compound, without the preliminary release of iodine. The absorbed-in iodotyrosines with blood flow are accessible to various tissues of an organism, the liver, first of all. The iodotyrosines in cells are subjected to different biochemical transformations. Fermentative deiodination releases free iodide to become accessible to synthetic processes in the thyroid gland. The dynamics of iodide entering the blood and the thyroid, using, as a source of iodine, its organic covalent bound form, differs in principle from that of the initial consumption of iodine in the form of its inorganic compounds. The iodotyrosines can be deaminated, decarboxylated, oxidized by the tyrosine ring, conjugated with glucosiduronic or sulfate groups. The formable conjugates can be extracted with bile. Iodinated metabolites are also brought out from an organism with urine.

It is hence only logical to see that iodine-containing amino acids with covalent bound iodine serve not only as an iodine source but also ensure self-regulation of iodine metabolism in an organism as being available for various biochemical transformations. Owing to this fact, the organism is capable of tolerating an excessive amount of iodine in the form of its organic compounds. Iodinated caseins both in a chemical nature of iodine-containing compounds and character of metabolism thereof are the analogs of natural iodinated compounds - main iodine source for humans. The high degree of protein iodination makes it possible to use BAA in extremely small amounts.

This example is a preferable alternative embodiment of the invention.

### Example 2

BAA to food, as a source of iodine, contains a tyrosine-iodinated soy protein together with elementary iodine iodinated starch. The soy protein is dissolved in water and iodination is carried out in a way similar to Example 1, with a lesser quantity of iodine (1.5-02 times) used, though. The soured starch solution (5%) is iodized with a 5% alcohol solution of elementary iodine. The iodine from such a combined BAA to food enters the organism in a differentiated manner: in covalent amino acid bound form and in the form of inorganic iodine. Despite a high degree of association of the iodine and starch, the latter releases a limited amount of iodine as a result of dissociation. The iodine reconstituted to iodide is admitted to blood. Thus, the combined BAA form to food will activate the alternative mechanisms of suction and iodine metabolism. This form facilitates a more rapid entry of iodine into the thyroid and iodine suction by independent biochemical mechanisms, a factor that is actual in case of pathology in the gastrointestinal tract.

### Example 3

BAA to food is obtained in the form of a combined preparation including a potassium iodate- inorganic compound of iodine and organic covalent bound iodine form-egg albumin iodinated in terms of tyrosine and histidine, which is synthesized in a way similar to Example I, with exclusion of thermal treatment, though, that is replaced with sterilizing microfiltration. Said BAA to food provides for enabling the alternative mechanisms of iodine suction. The iodate is sucked in the blood as iodide, albeit it is characterized by greater chemical stability.

### Example 4

BAA to food is prepared from hemoglobin food protein iodized hydrolyzates obtained under conditions corresponding to Example I and included in a gelatin capsule. The stabilizer used is represented by hyaluronic acid. The capsule has prolonged action. The preparation is mild, not affecting the mucosa. The capsules are convenient for administration, small in size, free of taste and smell.

### Example 5

Fat-soluble BAA to food is prepared in the form of a mixture of iodinated unsaturated fatty acids - linolenic, arachidonic, linoleic and iodinated isoprenes-carotenes and lycopin This BAA form is suitable for enriching rich-in-fat food products with iodine.

The BAA of interest has prolonged action. It is sucked, for example, as fatty acid in a limph, and stored in a fat depo. Iodine is released from these compounds as a result of fermentative catalysis. Advantages of said BAA reside in that covalent bound iodine may be administered in large doses, excluding a single release of free iodide in large amounts.

### Example 6

BAA to food, based on a mixture of iodinated protein hydrolyzates obtained from iodized egg albumin, and iodized isoprene-carotene is included in a vitamin-mineral complex. Said BAA provides an organism with organic iodine, enhances a biochemical effect of the vitamin-mineral complex. The normalized consumption of this iodine corresponds to generally accepted norms.

### Example 7

BAA to food of prolonged action is within a vitamin-mineral complex and is manufactured in the form of pills from a polymer matrix based on alginic acid. 4% alginic acid solution is added with an iodinated whey protein that is iodized in a way similar to Example I, a mixture of vitamins and microelements. An alginate solution is polymerized by introducing I M calcium chloride solution, moulded and dried. One pill contains 50 mcg of covalent bound organic iodine.

In the gastrointestinal tract, an alginate polymer matrix creates sterical hindrances to the fermentative hydrolysis of iodized protein and iodine-containing amino acids uptake. The polymer matrix prolongs iodine suction in the intestine.

### Example 8

BAA to food used is represented by iodinated amino acids - monoiodotyrosine and diiodotyrosine, which are distinguished by a very high iodine content - 41.4 and 58.7%, respectively. This degree of iodination, in spite of a limited solubility of these compounds in water, allows one to efficiently use them to enrich beverages. The small dimensions of molecules of iodine-containing material provide the efficient sterilization of iodinated beverages by ultrafiltration, kvass (a fermented drink), for example.

L-tyrosine (100 g) is dissoled in water, using ammonium solution. Tyrosine, as a phenolic derivative, is iodized with elementary iodine, with an alkali pH value maintained up to 9.5. The iodinated tyrosines are separated from an aqueous iodide solution by reextraction with butanol in the ratio of I : 3. The butanol solution of iodotyrosines is then dried on a rotary evaporator.

### Example 9

BAA to food used is represented by a goat milk iodized casein containing iodine in covalent bound form by analogy with mother's milk. This BAA is suitable for enriching mixtures for child's food of the "Malyutka" (baby) and "Malysh" (kiddy) kind. This is facilitated by a high degree of protein iodination and, hence, BAA meager quantities required for the enrichment of the mixtures and also a high thermal stability of the covalent iodine combination, good solubility and low allergenicity.

The technology of preparing iodinated infant formula includes the synthesis, according to Example I, of the iodized goat milk casein, introduction of iodized proteins into cow milk, homogenization of a milk base, vacuum thickening, and spray drying.

The efficiency of iodinated lactic proteins used, as an iodine source, for babies is obvious, because the only form of iodine in natural bringing up is represented by its covalent combinations with the proteins of mother's milk.

### Example 10

BAA to food - iodized peptides are obtained by a partial fermentative hydrolysis of the pre-iodized casein according to Example I. The hydrolysis of the iodized protein is carried out with trypsin or papain in a buffered solution with weak alkali pH values. This BAA has enhanced stability in a sequence of operations for the production of child's food or bakery products. In bread production, iodine-containing compounds are introduced into a batch before flour is added. Forming a true solution, iodine-containing molecules are further evenly distributed over a long loaf, which provides for the normalized consumption of iodine. An amount of BAA added to food - iodinated peptides depends on a degree of the iodine endemicity of a region.

### Example 11

Enrichment of meat products with compounds with covalent bound iodine for the purpose of the preventive treatment of iodine metabolism in the population is carried out by way of saturating the stuffing with iodized soy proteins. For an iodine-containing preparation to be distributed evenly, the stuffing is finely mixed with an iodized protein solution. An amount of the iodine-containing protein being added depends on iodine deficit offset norms established for a given locality. The synthetic iodized protein withstands heat treatment within meat products, cooked sausage, for example. The temperature of iodized tyrosine thermal decomposition is varied between 199 and 201°.

### Example 12

BAA to food, based on iodinated whey proteins is suitable for the iodine- enrichment of milk and lactic acid products. BAA synthesized in accordance with Example I excepting the fact that for iodination, use is made of preliminarily concentrated whey proteins, with a tyrosine content therein being one-half that of caseins, is desired to be added before the pasteurization of a milk base. The use of BAA in microquantities precludes its impact on the organoleptic properties of milk products. The degree of iodination of products is provided upon recommendations for a concrete region.

### Example 13

BAA to food in the form of dragee, based on iodized polypeptides is obtained by iodination of polypeptides, which are formed in extracting a heme from hemoglobins. Iodination of the purified polypeptides is conducted in accordance with Example I.

### Example 14

Fat-soluble BAA to food for the prophylaxis of iodine deficiency is prepared on the basis of iodized isoprene-carotene. Iodination is carried out in terms of unsaturated bonds. This BAA is included in the composition of a lipid-containing vitamin complex. The iodized carotene derivative entering an organism serves as an iodide source for the thyroid gland. The iodide is released as a result of fermentative catalysis.

### Example 15

The synthesis of iodized proteins is effected according to example I, with the preparation of a wet tablet mixture of an iodized protein solution (50 mcg of covalent bound iodine per tablet having a mass of 120 g), lactose, starch, magnesium stearate. Operations of mixing, granulating, drying, powdering and pressing the tablet mass are performed. The resultant tablet, 7 mm in dia. and 120 mg in mass, contains 50 mcg of physiologically efficient organic covalent bound iodine. BAA tablet form allows one to correct iodine metabolism individually, which form is convenient for use in field conditions. Said BAA form makes it possible to correct entry of iodine into an organism in relation to age-dependent and physiological peculiarities. BAA is suitable for long storage by virtue of the chemical stability of an organic covalent bound form of iodine.

### Example 16

The bioactive food additive used for the preventive treatment of iodine failure and optimization of iodine metabolism in animals is represented by an iodized protein of animal origin. The tyrosine-containing protein-hemoglobin is iodized chemically according to the run of Example I. Iodine is such compounds is in the covalent bound form of the 3-position of the phenolic ring of tyrosine amino acid residues. Also iodized are histidine amino acid residues. Iodination is carried out with no allowance for the condensation of iodized tyrosines to form thyroid hormonal compounds, for which purpose use is made of low temperatures (25-40°) and the limited time of iodination (4-6 hrs). BAFA contains iodine at high concentrations and is used in minor amounts. The degree of BAFA iodination is regulated by an amount of iodine introduced and the time of iodination. The iodine-containing BAFA is employed to enrich dry or liquid feeds.

BAFA is subjected to hydrolysis in the gastrointestinal tract, with iodine-containing amino acids freed. Iodine is sucked in the composition of these amino acids. Contrary to inorganic iodine compounds, the covalent bound iodine within amino said is involved in providing the most favourable iodine metabolism as making possible tissue deposition of iodoorganic compounds, chemical modification thereof, thus activating the mechanisms of excretion of iodine and iodoorganic matter from the organism. And last but not least, the iodinated amino acids represent an efficient iodine source as a result of tissue fermentative separation of iodide entering the thyroid gland. The iodized protein is a stable preparation, resistant to heat treatment, safe for animals. The degree of feed iodination is determined by age-dependent and physiological norms with reference to iodine provision in the organism.

### Example 17

BAFA is an egg albumin iodized protein hydrolyzate with covalent bound iodine, which is combined with organically bound iodine in non-covalent form, coupling with starch, for example. Coupling of iodine with the starch is a source of inorganic iodine and, most importantly, said iodine is released in the animal's bowels gradually and in certain limits. The covalent bound iodine and inorganic iodine are sucked through different mechanisms. Further metabolism thereof is different. This BAFA form accelerates the entry of iodine into the animal's thyroid gland.

Egg albumin solution is iodized according to the rum of Example I except a thermal treatment step. The iodized egg albumin solution is sterilized through microfiltration.

### Example 18

BAFA for the prevention of iodine failure is a combination of organically bound iodine - iodized soy protein and inorganic iodine compound - potassium iodide. BAFA enables the alternative mechanisms of iodine suction: in the composition of iodized amino acids of tyrosine and histidine and in the form of a free iodine-iodide anion. This BAFA is used in feeding domestic and farm animals and poultry within feeds.

### Example 19

BAFA for the prevention of iodine failure, comprising iodine in covalent bound form as iodinated unsaturated fatty acids - linoleic, linolenic and arachidonic in injectable purified olive oil is intended for providing animals with iodine in a grazing period in the fields. BAFA is administered in a subcutaneous or intramuscular manner in an amount of I to 2.5 mg/kg of live weight and has prolonged action to provide the animals with iodine for several months. The thus implanted BAFA exerts no toxic influence.

### Example 20

BAFA containing organic covalent bound iodine in the form of a mixture of an iodized soy protein hydrolyzate and iodinated isoprenes - lycopene and carotene is applicable for enriching premixes intended for feeding farm animals and poultry. BAFA features stability under technological conditions for preparing and storing the premixes. Isoprene derivatives are iodized in terms of unsaturated bonds.

### Example 21

Feed for laying hens comprises in 100 g 17 g raw protein, 11.72 MJ exchange energy and 3.2 g calcium; it is enriched with mineral components and vitamins.

The iodine source used is represented by an organic covalent compound - iodized egg albumin which is distinguished by a high tyrosine and histidine content. For iodination, use is made of hen egg albumin. The white is separated from the yolk and homogenized with an equal volume of 4% sodium chloride solution. Iodination is carried out with an iodine solution in a potassium iodide, maintaining the temperature and alkali pH value of the reaction mixture under continuous stirring. Completion of iodination is determined by the qualitative reaction for the presence of elementary iodine. The iodide is removed by the repeated precipitation of the iodized albumin using acid. The iodine-containing protein is resuspended with 2% sodium chloride. The iodide-free solution is subjected to sublimation or spray drying. Such iodination allows one to obtain a protein with more than 10% bound iodine. BAFA-dispersed protein is introduced with vitamins and mineral substances into a grain feed source (in the amount of 200 mcg per 100 g feed) to be agitated thoroughly.

Iodinated protein features stability within feeds. Iodine is admitted to a laying hen organism, as a component of non-hormonal iodine-containing acid. A diodinase-tissue ferment splits off the iodine at the 3- and 5-positions of the tyrosine phenolic ring to provide the thyroid gland with the required iodide. The given example is a preferable alternative embodiment of the invention.

### Example 22

BAFA is manufactured in the form of tables or powders as a component of vitamin-mineral complexes for animals and poultry. The iodine-containing compounds used are represented by water-soluble polypeptide iodine-containing hydrolyzates. This BAFA increases the efficiency of vitamin-mineral complexes.

### Example 23

BAFA prolonged form represents an iodine-containing water-soluble material-iodinated soy proteins included in a firm dried agarose gel. BAFA thermal stability makes it possible to introduce it into an agarose suspension and obtain melts.

An agarose polymer matrix sterically hinders BAFA fermentative hydrolysis in the gastrointestinal tract and iodine-containing amino acid uptake from the polymer matrix. This property of the preparation prolongs a process of iodine suction in an animal body.

### Example 24

BAFA water-soluble form - iodinated protein hydrolyzates from whey proteins are suitable for enriching feeds for younger farm animals, calves and young pigs, for example. The use of liquid feeds, such as milk substitutes, enables one to provide the normalized consumption of iodine by the animals and facilitate entry of iodine into the organism in the most adequate form.

### Example 25

BAFA for the prevention of iodine failure containing iodine in covalent bound form, representing an iodized tyrosine solution is intended for providing animals with iodine in a grazing period with the aid of subcutaneous implantation. The implantat containing diiodotyrosine synthesized according to the example, is administered into cattle under the skin in the amount of I mg/kg of live weight in terms of bound iodine. The preparation in the form of an implantat provides for a long (3 months) entry of iodine into the thyroid gland without exerting a negative influence on the animal body. The preparation promotes an additional gain in weight of 5 to 10 kg with no above-the-standard feed costs.

### Example 26

Fat-soluble BAFA representing an iodized unsaturated fatty acid - arachidonic - is used for introduction into a vitamin complex based on plant oil. Iodination of said arachidonic acid is carried out in terms of unsaturated bonds.

### Example 27

According to Example I, there is conducted the synthesis of a bioactive additive to food - iodized goat milk proteins without a step of preservation. Upon purification of a protein solution from iodides, the preparation is subjected to sublimation drying. The ultimate humidity of the preparation is at least 3.5%. The goat milk proteins are characterized by a lower level of allergenicity in comparison with cow proteins. BAA is included in the composition of a combined complex comprising vitamins and mineral substances. One capsule contains vitamin B₁ - 5 mg ; vitamin B₆ - 2 mg ; vitamin B₁₂-1.5 mg ; nicotinamide - 5 mg, calcium pantothenate - 2 mg ; ascorbic acid - 25 mg ; iron - 5 mg ; copper - 100 mcg : zinc - 100 mcg : magnesium - 3.5 mg ; calcium - 15 mg ; phosphorus - 10 mg ; cobalt - 100 mcg; manganese - 500 mcg; molybdenum - 200 mcg; organic covalent bound iodine - 100 mcg.

One capsule of a complex is taken daily for the prophylaxis and treatment of vitamin and mineral deficiency in the organism.

## Claims

1. Biologically active additive to food (BAA) for the preventive treatment of iodine deficiency, comprising an organic iodine-containing substance, **characterized in that** said organic iodine-containing substance is a synthetic organic compound with covalent bound iodine, selected from the group consisting of carboxylic acids, unsaturated fatty acids, lipids, terpenes, alkanes, terpenoids, isoprenes, peptides, polypeptides, amino acids, protein hydrolyzates, polypeptide hydrolyzates, proteins of plant, animal, microbiological origin, a mixture of lipids and unsaturated fatty acids, a mixture of isoprenes and terpenes, a mixture of isoprenes and protein hydrolyzates, a mixture of isoprenes and unsaturated fatty acids, a mixture of proteins of plant, animal and microbiological origin, a mixture of proteins of plant and animal origin, a mixture of proteins of animal and microbiological origin, a mixture of proteins of plant and microbiological origin, and in the proteins, peptides, polypeptides, amino acids, polypeptide and protein hydrolyzates, the iodine is covalent bound at the 5- and 3-positions or at the 3-position of the phenolic ring, the amino acids and proteins being selected from the group of amino acids and proteins having no hormonal thyroid activity, and being obtained under conditions precluding the condensation of tyrosine kernels.

2. BAA to food according to claim I, **characterized in that** the proteins of animal origin used are represented by caseins, egg albumin, lactic or whey proteins, hemoglobins while the proteins of plant origin - soy protein.

3. BAA to food according to claim I or 2, **characterized in that** it further contains an organic compound with non-covalent bound iodine.

4. BAA to food according to any one of claims I to 3, **characterized in that** it further contains inorganic iodine.

5. BAA to food according to any one of claims I to 4, **characterized in that** it represents prolonged form.

6. BAA to food according to any one of claims I to 5, **characterized in that** it is implemented in the form of tablets, powders, capsules, microcapsules, dragee, solutions.

7. BAA to food according to claim 6, **characterized in that** the microcapsules are prepared by microincapsulation using carbohydrate, protein or lipid raw material.

8. BAA to food according to claims I to 7, **characterized in that** the iodine-containing substance is included in an inert polymer matrix from carrageenan, pectin, agarose or uronic acids, for example.

9. Vitamin-mineral complex, **characterized in that** it includes the BAA to food of any one of claims 1 to 8.

10. Foodstuff containing BAA to food, **characterized in that** BAA contained therein is represented by BAA to food according to claims I to 9.

11. Foodstuff according to claim 10, **characterized in that** it represents milk product.

12. Foodstuff according to claim 10, **characterized in that** it represents lactic acid product.

13. Foodstuff according to claim 10, **characterized in that** it represents meat product.

14. Foodstuff according to claim 10, **characterized in that** it represents bakery product.

15. Foodstuff according to claim 10, **characterized in that** it represents bread.

16. Foodstuff according to claim 10, **characterized in that** it represents drink.

17. Foodstuff according to claim 10, **characterized in that** it represents juice.

18. Foodstuff according to claim 10, **characterized in that** it represents confectionery.

19. Foodstuff according to claim 10, **characterized in that** it represents infant formula product.

20. Biologically active food additive (BAFA) for the preventive treatment of iodine deficiency, comprising an organic iodine-containing substance, **characterized in that** the organic iodine-containing substance represents a synthetic organic compound with covalent bound iodine, selected from the group consisting of carboxylic acids, unsaturated fatty acids, lipids, terpenes, alkanes, terpenoids, isoprenes, peptides, polypeptides, amino acids, protein hydrolyzates, polypeptide hydrolyzates, proteins of plant, animal, microbiological origin, a mixture of lipids and unsaturated fatty acids, a mixture of isoprenes and terpenes, a mixture of isoprenes and protein hydrolyzates, a mixture of isoprenes and unsaturated fatty acids, a mixture of proteins of plant, animal and microbiological origin, a mixture of proteins of plant and animal origin, a mixture of proteins of animal and microbiological origin, a mixture of proteins of plant and microbiological origin, and in the proteins, peptides, polypeptides, amino acids, polypeptide and protein hydrolyzates, the iodine is covalent bound at the 5- and 3-positions or at the 3-position of the phenolic ring, the amino acids and proteins being selected from the group of amino acids and proteins having no hormonal thyroid activity, and being obtained under conditions precluding the condensation of tyrosine kernels.

21. BAFA according to claim 20, **characterized in that** the animal origin proteins used are represented by caseins, egg albumin, lactic or whey proteins, hemoglobins and the plant origin proteins - soy protein.

22. BAFA according to any one of claims 20 and 21, **characterized in that** it further contains an organic compound with non-covalent bound iodine.

23. BAFA according to any one of claims 20 to 22, **characterized in that** it further contains inorganic iodine.

24. BAFA according to any one of claims 20 to 23, **characterized in that** it represents prolonged form.

25. BAFA according to any one of claims 20 to 24, **characterized in that** it is implemented in the form of tablets, powders, capsules, microcapsules, dragee, solutions, injectable solutions.

26. BAFA according to claim 25, **characterized in that** the microcapsules are manufactured by microincapsulation using carbohydrate, protein or lipid raw material.

27. BAFA according to any one of claims 20 to 26, **characterized in that** the iodine-containing substance is included in an inert polymer matrix of, for example, carrageenan, pectin, agarose or uronic acids.

28. BAFA according to any one of claims 20 to 27, **characterized in that** it represents premix.

29. BAFA according to any one of claims 20 to 27, **characterized in that** it is within premixes.

30. BAFA according to any one of claims 20 to 27, **characterized in that** it is within vitamin-mineral complexes.

31. BAFA according to any one of claims 20 to 30, **characterized in that** it is within feeds.

32. BAFA-containing food product, **characterized in that** BAFA contained therein is represented by BAFA according to any one of claims 20 to 30.
